# EUROPÄISCHE PATENTSCHRIFT

(11) **EP 4 211 697 B1**
(45) Veröffentlichungstag und Bekanntmachung des Hinweises auf die Patenterteilung: **08.04.2026**
(21) Anmeldenummer: 21766161.0
(22) Anmeldetag: 24.08.2021
(51) Int. Cl.: A61M 5/14, A61M 5/142, A61M 5/145, A61M 5/172, A61M 5/20, G16H 20/17, G16H 40/63

(54) **VERABREICHUNGSSYSTEME UND METHODEN ZU DEREN NUTZUNG**
DELIVERY SYSTEMS AND METHODS FOR THEIR USE
SYSTÈMES DE DISTRIBUTION ET MÉTHODES D'UTILISATION

(30) Priorität: 09.09.2020 CH 11232020
(43) Veröffentlichungstag der Anmeldung: 19.07.2023
(73) Patentinhaber: mylife Diabetes Care AG, 3400 Burgdorf (CH)
(72) Erfinder: SELMAN, Hasan, 4562 Biberist (CH); BOSSHARD, David, 3400 Burgdorf (CH); KRÄMER, Christian, 4571 Lüterkofen-Ichertswil (CH); EBENER, Stefan, 3018 Bern (CH)
(74) Vertreter: Finklenburg, Susanne
(86) Internationale Anmeldenummer: PCT/EP2021/073390
(87) Internationale Veröffentlichungsnummer: WO 2022/053307

(56) Entgegenhaltungen:
- EP-A1- 3 660 856
- US-A1- 2018 015 218
- US-A1- 2019 378 603

## Beschreibung

### TECHNISCHES GEBIET

Die vorliegende Erfindung betrifft das Gebiet Verabreichungssysteme zur Verabreichung von fluiden Medikamenten mittels einem Verabreichungsgerät, insbesondere einer Infusionspumpe oder einem Injektionsgerät. Speziell betrifft die vorliegende Erfindung auch die drahtlose Kommunikation innerhalb der Verabreichungssysteme und Softwarefunktionen in den Verabreichungssystemen.

### HINTERGRUND DER ERFINDUNG

Vorrichtungen zur Verabreichung von fluiden Medikamenten sind dem Fachmann aus der internationalen Patentklasse A61M5 hinlänglich bekannt.

Aufgrund der steigenden Gesundheitskosten in den entwickelten Ländern wird die Selbstverabrei-chung von fluiden Medikamenten, auch wenn diese injiziert oder infundiert werden müssen, immer wichtiger. Da Patienten und Patientinnen in den meisten Fällen keine medizinischen Fachpersonen sind, besteht die Gefahr von Fehlmanipulationen oder falscher Anwendung von falschen Medika-menten.

Ein typisches Beispiel ist die Selbstverabreichung von Insulin bei Diabetespatienten. Insulin wird in Spritzen, Injektionspens oder auch mit Infusionspumpen verabreicht. Es gibt viele verschiedene In-suline in unterschiedlichen Konzentrationen und unterschiedlichen Wirkungsprofilen. Ein häufiges Primärpackmittel von Insulinen in flüssiger Formulierung ist die Karpule, entweder als 3ml oder 1.6 ml Version. Alternativ gibt es vorgefertigte Einweg Pens, in welche die entsprechende Insulinkarpule bereits eingebaut ist.

Es besteht also auf einer Seite die Gefahr, dass Diabetespatienten die falsche Form verabreichen mit potentiell gravierenden Folgen, wenn zum Beispiel statt der täglichen Dosis langsam wirksames In-sulin (Sanofi Lantus) die gleiche Anzahl Einheiten sehr schnell wirksames Insulin (Novo Nordisk Fiasp) verabreicht wird, kann zu einer Unterzuckerung und einem folgenden Kollaps des Diabetespa-tienten kommen.

Auf der anderen Seite sollten Patienten und Patientinnen nicht bevormundet werden und sollen selber entscheiden können, wann er oder sie sich welches Medikament verabreicht. Die potentielle negative Folge einer Bevormundung ist eine sinkende Adhärenz der Patienten und Patientinnen an eine bestimmte Therapie.

Es ist weiter im Falle von Infusionspumpen so, dass diese insbesondere bei Insulinpumpen für bestimmte Insulinprodukte getestet und freigegeben sind. Hier besteht die Gefahr von ungenauer Dosierung, wenn ein vermeintlich gleiches Produkt in vermeintlich gleichgrosser Karpule verwendet wird.

Es besteht ein Bedürfnis die Sicherheit bei der Verabreichung von fluiden Medikamenten weiter zu erhöhen, ohne dass dabei eine Bevormundung der Patienten und Patientinnen entstehen könnte, die der Therapieadhärenz abträglich wäre.

Der Begriff fluides "Produkt", fluides "Medikament" oder fluide "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Die US2018/0015218 A1 offenbart Methoden zum Pairing von einem portablen Gerät mit einem medizinischen Gerät.

Die EP 3660856 A1 offenbart Methoden, welche die Nutzung von Medikamentenverabreichungs-vorrichtungen unterstützen, sog. Augmented Reality-Methoden. Dabei wird mit der Kamera eines Mobilgeräts mindestens ein Marker (der Medikamentenverabreichungsvorrichtung oder deren Ver-packung) fotografiert. Die Bilddaten werden sodann an einen Anbieter gesendet, welcher seinerseits Daten zurücksendet, welche die Nutzung der Medikamentenverabreichungsvorrichtung unterstützt.

Die US2019/0378603 A1 offenbart einerseits automatisches Pairing zwischen einem computerartigen Gerät mit Kamera und einem medizinischen Gerät. Das Dokument offenbart weiter, dass in einigen Ausführungsformen das Funktionieren des Verabreichungsgerätes an das computerartige Gerät bindbar sei.

### DARSTELLUNG DER ERFINDUNG

Es ist eine Aufgabe der Erfindung, Verabreichungssysteme zumindest bestehend aus einen mobilen Kommunikationsgerät und einem Verabreichungsgerät sowie zugehörige Methoden bereitzustellen, welche der benutzenden Person eine erhöhte Sicherheit bei der Nutzung des Systems bieten.

Die Aufgabe wird gelöst durch die in den unabhängigen Ansprüchen definierten Systeme und Methoden. Vorteilhafte Weiterentwicklungen ergeben sich aus den abhängigen Ansprüchen, sowie Beschreibung und Zeichnungen.

In einer Ausgestaltung der Erfindung besteht ein erfindungsgemässes Verabreichungssystem zur Verabreichung eines fluiden Medikamentes zumindest aus einem Verabreichungsgerät und einem mobilen Kommunikationsgerät. Die beiden Geräte können drahtlos und bidirektional miteinander kommunizieren.

Auf dem Kommunikationsgerät, welches zum Beispiel ein Smart Phone wie ein iPhone sein kann, ist Software oder insbesondere eine Software-Applikation, installiert, welches mehrere Funktionen umfasst, welche der Kommunikation mit und/oder der Steuerung des Verabreichungsgeräts dienen. Auf dem Kommunikationsgerät sind mehrere Kommunikationsvorrichtungen vorhanden, welche durch die Software genutzt werden könnten. Beispielhaft kann es sich dabei um Vorrichtungen für Mobilfunk, WLAN, Bluetooth, NFC, ZigBee, Z-Wave und/oder ähnliche handeln. Das Kommuni-kationsgerät umfasst weiter eine Kamera, welche von der Software angesprochen und gesteuert werden kann. Auch umfasst das Kommunikationsgerät typische weitere Elemente wie eine Benutzer-schnittstelle in Form eines Bildschirms und weiteren Bedienelementen, Tasten, oder eines Berüh-rungssensitiven Bildschirms, Speicher, zum Abspeichern von Daten, sowie eine elektronische Steu-erung mit mindestens einem Prozessor, einem Betriebssystem sowie weitere optionale Elemente wie Sensoren.

Das Verabreichungsgerät, umfasst ein Reservoir mit dem Medikament, welches austauschbar ist, sowie eine Ausschüttvorrichtung. Das heisst, zur Verabreichung des Medikaments wird mit Hilfe der Ausschüttvorrichtung Medikament aus dem Reservoir verabreicht. Wenn das Reservoir leer ist, kann es mit einem neuen vollen Reservoir ersetzt werden. Weiter verfügt das Verabreichungsgerät über eine elektronische Steuerung und einer mit der Steuerung verbundenen Kommunikationsvor-richtung zur drahtlosen Kommunikation mit dem Kommunikationsgerät. Die möglichen, dabei zur Anwendung gelangenden Technologien wurden bereits erwähnt.

Beim Reservoir kann es sich um verschiedenste dem Fachmann bekannte Formen der Primärpack-formen für fluide Medikamente handeln, hier seien beispielhaft und nicht abschliessend Beutel, Fla-schen, Kartuschen, Karpulen, Spritzen oder spritzenähnliche Reservoire erwähnt. Erfindungsgemäss ist auf einer äusseren Oberfläche des Reservoirs oder dessen Verpackung eine Information angeordnet.

Diese Information kann mit Hilfe der Kamera von der Software gelesen und ausgewertet werden. Diese Information kann erfindungsgemäss dazu verwendet werden, um in der Software implementierte Funktionen zu aktivieren, zu deaktivieren, respektive deaktiviert zu halten. Diese Funktionen können der Kommunikation oder der Steuerung des Verabreichungsgeräts dienen.

Das Lesen der Information mit der Kamera findet vorteilhaft beim Wechseln des Reservoirs statt. Alternativ, wenn mehrere Reservoirs in einer Verpackung sind, kann das Lesen auch beim Anbre-chen einer neuer Verpackung stattfinden. In einer weiteren Alternative, kann das Lesen der Informa-tion zeitgesteuert sein, so dass beim Wechseln des Reservoirs nach einem bestimmten Zeitraum die Information gelesen wird, zum Beispiel nach einer Woche oder einem Monat.

Die Information ist dabei vorteilhaft gut maschinenlesbar ausgestaltet, zum Beispiel als Barcode, Data Matrix Code, QR Code, maschinenlesbare Schrift, oder maschinenlesbares Muster.

Die Information kann verschiedene Elemente umfassen, wobei hier beispielhaft und nicht abschliessend folgende Elemente erwähnt werden:
- eine eindeutige Produktkennung,
- eine Chargenummer oder eine Lotnummer,
- eine Seriennummer,
- ein Verfallsdatum,
- ein Herstell- oder Produktionsdatum,
- eine Herstellerkennung.

Beim Verabreichungsgerät kann es sich in vorteilhaften Ausführungsformen der Erfindung um eine Infusionspumpe, insbesondere um eine Insulinpumpe, eine Patch Pumpe, oder ein Injektionsgerät handeln. Unter dem Begriff Injektionsgerät wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionsgerät im die Injektionsnadel nicht über einen längeren Zeitraum von Stunden im Gewebe. Dies im Unterschied zur Infusionspumpe oder der Patch Pumpe, bei welchen die Infusionskanüle bis zu mehreren Tagen oder gar einer Woche im Gewebe verbleiben kann. Bei der klassischen Infusionspumpe ist die Infu-sionskanüle Teil eines externen Infusionssets, bei der Patch Pumpe ist die Infusionskanüle Teil einer kurzen Infusionsleitung, welche häufig innerhalb der Patch Pumpe angeordnet und wobei nur die Kanüle aus dem Gehäuse heraus ins Gewebe ragt.

In weiteren Ausgestaltungen der Erfindungen ist die Information als genormter DataMatrix Code oder Barcode ausgestaltet, insbesondere nach der ISO/IEC 15418 und/oder ISO/IEC 16022. Vorteil-haft handelt es sich dabei um GS1 Anwendungsidentifikationen (https://www.gs1.org/). Vorteilhaft sind diese Ausgestaltungen auch konform mit der Fälschungsschutzrichtlinie 2011/62/EU der Euro-päischen Union oder analogen Vorgaben von US Behörden, wie der FDA, oder anderen offiziellen Instanzen.

In einer vorteilhaften Ausgestaltung umfasst ein erfindungsgemässer DataMatrix Code, insbesondere ein GS1 DataMatrix Code, eine nationale oder internationale Artikelnummer (insbesondere eine sogenannte NTIN oder eine GTIN), ein Verfallsdatum, eine Chargen- oder Lotnummer und optional eine Seriennummer. Unter NTIN wird eine ,National Trade Item Number' und unter GTIN eine ,Global Trade Item Number' verstanden.

Erfindungsgemäss sind auch Methoden zur Freigabe der erwähnten Funktionen der Software.

In einer erfindungsgemässen Ausführungsform einer Methode, wird, insbesondere beim Wechseln des Reservoirs, die Information durch die Kamera. Die durch die Kamera erfasste Bildinformation wird im Kommunikationsgerät zwischengespeichert. Anschliessend kann die Information mit Hilfe des Prozessors aus der Bildinformation extrahiert und zur weiteren Verwendung durch die Software gespeichert werden. In einem weiteren Schritt können Teile der Information oder die ganze Informa-tion über eine der Kommunikationsvorrichtungen des Kommunikationsgeräts, insbesondere über Mobilfunk oder WLAN, an einen entfernten Server gesendet werden. Der Begriff Server soll im Kontext des vorliegenden Dokumentes breit ausgelegt werden. Bei einem Server kann es sich um eine virtuelle Instanz einer Serverfarm oder eines privaten oder öffentlichen Cloud-Dienstes handeln, oder es kann sich um eine virtuelle Instanz eines einzelnen Servers oder direkt um einen oder mehrere Server, wobei dem Fachmann weitere Möglichkeiten offenbar sind. Der Server umfasst neben den typischen Elementen, welche einen Servercomputer auszeichnen, eine Datenbank, welche eine Über-prüfung der vom Server empfangenen Information erlaubt. Die Datenbank enthält dazu Informatio-nen zu den aktivierbaren Funktionen sowie Referenz-Information, welche die empfangene Informa-tion mit den aktivierbaren Funktionen in Verbindung bringen kann. Die Überprüfung hat die Funk-tion, zu prüfen, ob und/oder welche aktivierbaren Funktion mit der empfangenen Information kompatibel ist. Nachdem der Server eine Überprüfung durchgeführt hat, wird auf dem Server eine Ant-wort an das Kommunikationsgerät mit einem Resultat der Überprüfung erstellt und an dieses gesendet. Die Software wertet so dann die Antwort aus. In der Folge wird oder werden entweder keine der aktivierbaren Funktionen, ein Teil der aktivierbaren Funktionen oder alle aktivierbaren Funktionen freigegeben. Alternativ können auch eine oder mehrere aktivierbare Funktionen deaktiviert werden. So kann sichergestellt werden, dass nur solche aktivierbare Funktionen aktiviert sind, welche auch zur Information passen.

In einer vorteilhaften Ausgestaltung der Ausführungsform der Methode werden diejenigen aktivierbaren Funktionen freigegeben, welche gemäss Antwort mit der Information passen, insbesondere kompatibel, sind. In einer vorteilhaften Ausgestaltung enthält die Antwort des Servers eine Liste mit den kompatiblen aktivierbaren Funktionen. Alternativ, kann eine solche Liste auch die inkompatiblen aktivierbaren Funktionen enthalten und es werden entsprechend diejenigen aktivierbaren Funk-tionen aktiviert, die nicht auf der Liste sind. Um die Datenmenge in der Antwort möglichst klein halten zu können, kann die Liste auch komprimiert sein oder codiert sein, insbesondere kann die Liste mittels einer Hash-Funktion codiert werden. Andere Varianten der Datenmengenoptimierung sind dem Fachmann offensichtlich und weichen nicht vom erfinderischen Grundgedanken ab.

In einer alternativen Ausführungsform der Methode, welche eine Abwandlung der oben beschriebenen Methode und/oder deren Weiterentwicklungen ist, geschieht die Überprüfung der Information nicht auf einem entfernten Server, sondern auf dem Kommunikationsgerät. Bei dieser alternativen Ausführungsform der Methode wird die Datenbank lokal auf dem Kommunikationsgerät vorgehalten. In einer vorteilhaften Weiterentwicklung dieser Methode, wird die Datenbank in regelmässigen oder unregelmässigen zeitlichen Intervallen über eine der Kommunikationsvorrichtungen des Kom-munikationsgeräts auf einen neuen Stand gebracht.

In einer weiteren alternativen Ausführungsform der Methode, welche eine Abwandlung der alternativen Ausführungsform der Methode ist, geschieht die Überprüfung der Information zumindest Teil-weise auf dem Verabreichungsgerät.

In einer vorteilhaften und erfindungsgemässen Weiterentwicklung der oben beschriebenen Ausfüh-rungsformen kann die Überprüfung ergeben, dass eine der aktivierbaren Funktionen nicht zur Infor-mation passt, insbesondere nicht kompatibel ist, wodurch in diesen Weiterentwicklungen sodann keine der aktivierbaren Funktionen aktiviert wird.

In einer weiteren vorteilhaften Weiterentwicklung umfasst das Kommunikationsgerät weiter eine Echtzeituhr und einen damit verbundenen Kalender, wenn nun die Information wie oben beschrieben ein Verfallsdatum und/oder ein Herstelldatum umfasst, kann die Überprüfung weiter die Überprü-fung des Datums umfassen und das Resultat die Aktivierung der aktivierbaren Funktionen beeinflussen.

Wird festgestellt, dass das Verfallsdatum bereits erreicht oder überschritten ist (das Medikament gilt als abgelaufen), kann die Software eine Meldung an die Benutzerschnittstelle ausgeben, in welcher eine benutzende Person vor der Benutzung des Medikamentes gewarnt wird und so die Sicherheit der Benutzung des Systems verbessert werden. Dabei kann das Verfallsdatum auch über das Her-stelldatum und eine bekannte maximal zulässige Lagerdauer bestimmt werden.

Als weitere Erhöhung der Sicherheit kann bei verfallenem Medikament die Software dazu genutzt werden, einen Steuerbefehl an das Verabreichungsgerät zu senden, mit welchem die Verabreichung von Medikament temporär gestoppt oder blockiert wird. Damit soll nicht die Nutzung eines abgelaufenen Medikaments komplett unterbunden werden. Die benutzende Person kann manuell die Sperrung des Verabreichungsgeräts aufheben. Dies im Bewusstsein, ein abgelaufenes Medikament zu nutzen.

Bei den aktivierbaren Funktionen kann es sich um eine breite Palette von Funktionen der Software handeln. Um das Verabreichungssystem sicher nutzen zu können, sollte die benutzende Person nur Medikamente nutzen, welche gültige Information aufweisen. Dies wird weiter unten in der Figurenbeschreibung noch weiter ausgeführt. Die Palette kann eine oder mehrere der folgenden Funktionen aufweisen, wobei die Aufzählung keineswegs abschliessend ist:
- Fernsteuerungsfunktion, bei welchen die Software Steuerbefehle an das Verabreichungsgerät sendet und damit das Kommunikationsgerät die Rolle einer Fernsteuerung übernehmen kann:
   ∘ Bolus-Programmierung und -Befehl (also die Abgabe einer einzelnen Medikamentendosis), wobei sich um einen Sofortbolus (welcher in einer einzelnen Ausschüttung abgegeben wird), einen verzögerten Bolus (welcher in mehreren einzelnen Ausschüttungen über eine vorgegebene Dauer abgegeben wird) oder um einen Kombinationsbolus (Kombination aus einem Sofortbolus und einem verzögerten Bolus) handeln kann. Der Bolus wird in der Software bezüglich den Abgabeparametern programmiert und dann an das Verabreichungsgerät gesendet. Das Verabreichungsgerät empfängt den Befehl und führt ihn aus.
   ∘ Starten, Stoppen, Pausieren der Medikamentenabgabe
   ∘ Temporäres reduzieren der Medikamentenabgabe
   ∘ Ändern von Betriebsparametern auf dem Verabreichungsgerät (wie maximale tägliche Dosis oder maximale Dosisgrösse)
- Convenience-Funktionen in Software wie ein Bolusrechner, der auf Basis von Eingabewerten eine Bolusempfehlung berechnet
- Automatische Dosisanpassungsalgorithmen, wenn zum Beispiel das Verabreichungssystem ein Diabetes Management System ist, mit einem kontinuierlichen Blutzuckermessgerät, einem Kommunikationsgerät und einem insulin-Verabreichungsgerät, und bei welchem das Kommunikationsgerät die Rolle des closed-loop controllers übernimmt. Dabei empfängt das Kommunikationsgerät Daten vom kontinuierlichen Messgerät und passt Insulinverabreichungsrate automatisch auf Basis von Dosisdaten und Blutzuckerdaten an.

Folgend wird eine alternative Ausführungsform des Verabreichungssystems beschrieben, wobei wichtig ist festzuhalten, dass die obig beschriebenen Methoden erfindungsgemäss auch für diese Ausführungsform Anwendung finden können. Im Folgenden werden vor allem die Unterschiede zur ersten beschriebenen Ausführungsform des Systems beschrieben, ansonsten gilt die Offenbarung analog auch für diese alternative Ausführungsform des Systems.

In dieser alternativen Ausführungsform des Systems besteht das Verabreichungsgerät aus einem Injektionsgerät und einem Zusatzmodul, kurz Add-on genannt. Injektionsgerät und Add-on sind im Gebrauch operativ, insbesondere mechanisch, miteinander lösbar verbunden. Wichtig ist, dass das Add-on zeitlich gestaffelt mit mehreren oder auch verschiedenen Injektionsgeräten genutzt werden kann. Das Injektionsgerät enthält ein Medikamentenreservoir, welches mittels einer ebenfalls vorhandenen Ausschüttvorrichtung abgegeben werden kann. Beim Injektionsgerät kann es sich um einen Injektionspen, einen sogenannten Patch-Injektor oder einen sogenannten Autoinjektoren handeln. Bevorzugt handelt es sich beim Injektionsgerät um ein Einweggerät, welches über eine oder mehrere Dosisabgaben entleert wird und anschliessend fachgerecht entsorgt werden kann. Vor dem ersten Gebrauch wird das Add-on mit dem Injektionsgerät verbunden und nach der letzten Verwendung wieder getrennt, wodurch das Add-on wieder bereit für ein neues Injektionsgerät ist.

Im Unterschied zur ersten beschriebenen Ausführungsform des Systems ist bei dieser alternativen Ausführungsform des Systems die Information bevorzugt auf einer äusseren Oberfläche des Injektionsgerätes aufgebracht. Alternativ kann die Information auch auf einer Verpackung von mehreren identischen Injektionsgeräten angeordnet sein, zum Beispiel auf einer Packung Insulinpens. Alternativ, wenn das Reservoir fest mit dem Injektionsgerät verbunden ist, das heisst, wenn Reservoir und Injektionsgerät nur destruktiv getrennt werden können, kann die Information nach wie vor auch auf einer äusseren Oberfläche des Reservoirs angeordnet sein. Das setzt allerdings voraus, dass die Information noch sichtbar ist, wenn das Reservoir am oder im Injektionsgerät an- oder eingebaut ist.

Das Add-on verfügt eine über eine elektronische Steuerung und einer mit der Steuerung verbundenen Kommunikationsvorrichtung zur drahtlosen Kommunikation mit dem Kommunikationsgerät. Weiter umfasst das Add-on einen oder mehrere Sensoren, die mit der Steuerung verbunden sind. Damit können, wenn das Add-on mit dem Injektionsgerät verbunden ist, verschiedene Zustände des Injektionsgerätes detektiert werden und zum Beispiel über die Kommunikationsvorrichtung an das Kommunikationsgerät gesendet werden.

Das Add-on umfasst weiter ein Gehäuse, welches die Steuerung, den mindestens einen Sensoren sowie die Kommunikationsvorrichtung zumindest teilweise umschliesst. Bevorzugt umfasst das Gehäuse eine Aufnahme, über welche das Injektionsgerät mit dem Add-on mechanisch verbunden werden kann. Beispielhaft kann im Gehäuse eine etwa zylindrische Vertiefung vorgesehen sein, in welche ein Ende eines Injektionspens oder eines Autoinjektors eingeschoben werden kann. Wie erwähnt ist die Verbindung zwischen Add-on und Injektionsgerät bevorzugt auch mechanisch und es können an Injektionsgerät und/oder Add-on passende Halteelemente vorgesehen sein, welche eine unbeabsichtigte Trennung verhindern. Dies können insbesondere Gewindeelemente oder Schnappelemente sein.

Die erfindungsgemässen Methoden wie oben beschrieben funktionieren mit dieser alternativen Ausführungsform des Systems analog oder gleich.

Ist ein Injektionsgerät fertig gebraucht, so wird es vom Add-on getrennt und ein neues Injektionsgerät wird eingesetzt. Vor, während oder auch nach dem Ersetzen wird die Information, welche zum neuen Injektionsgerät gehört von der Kamera erfasst und folgend wie beschrieben geprüft.

In einer weiteren Abwandlung der ersten Ausgestaltung der Erfindung und der mit der ersten Ausgestaltung verbundenen Varianten wird folgend eine weitere Variante beschrieben (basierend auf der obigen Beschreibung). In dieser weiteren Variante handelt es sich beim Verabreichungsgerät um eine Infusionspumpe, insbesondere um eine Insulinpumpe. Das Reservoir ist bei dieser weiteren Variante vorzugsweise eine handelsübliche Karpule, auf welche die Information aufgedruckt ist. Bei dieser weiteren Variante wird die Information erst dann durch die Kamera erfasst wird, wenn das Reservoir in die Infusionspumpe eingesetzt ist. Damit das funktioniert, umfasst die Infusionspumpe ein Fenster, durch welches das Reservoir und die Information auf dem Reservoir sichtbar sind. Mit der Kamera wird sodann nicht nur die Information erfasst, sondern es wird auch Bildinformation zur Pumpe erfasst. Die Bildinformation kann zum Beispiel Form- und/oder Farbinformationen der Infusionspumpe umfassen. Mit Bilderkennungssoftware, welche auf dem Kommunikationsgerät oder einem im System eingebundenen Server installiert ist, kann so zum Beispiel der Typ der Insulinpumpe erkannt werden. Die so aus der Bilderkennung gewonnene Bildinformation kann *zusammen* mit der Information erfindungsgemäss dazu verwendet werden, um in der Software implementierte Funktionen zu aktivieren, zu deaktivieren, respektive deaktiviert zu halten. Diese Funktionen können der Kommunikation oder der Steuerung des Verabreichungsgeräts dienen. Denkbar und ebenfalls erfindungsgemäss können Information und Bildinformation auch dazu verwendet werden um separate Funktionen zu aktivieren oder zu deaktivieren. Diese weitere Variante ist mit den oben beschriebenen Ausführungsformen in Arten und Weisen, wie sie dem Fachmann zugänglich sind, beliebig kombinierbar, ohne vom Erfindungsgedanken abzukommen.

Die Bildinformation kann in einer alternativen Ausführung dieser weiteren Variante auch Codes oder Text enthalten, welche auf der Oberfläche der Pumpe angeordnet sind. Zum Beispiel kann es sich um die Seriennummer der Pumpe handeln

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
- Fig. 1: zeigt ein erfindungsgemässes Beispielsystem
- Fig. 2a: zeigt ein zweites erfindungsgemässes Beispielsystem
- Fig. 2b: zeigt Add-on und Injektionsgerät in zusammengebauten Zustand
- Fig. 3: zeigt ein weiteres Beispiel für ein erfindungsgemässes Verabreichungsgerät, welches alternativ in das erfindungsgemässe System der Figur 1 eingebettet sein kann

### FIGURENBESCHREIBUNG

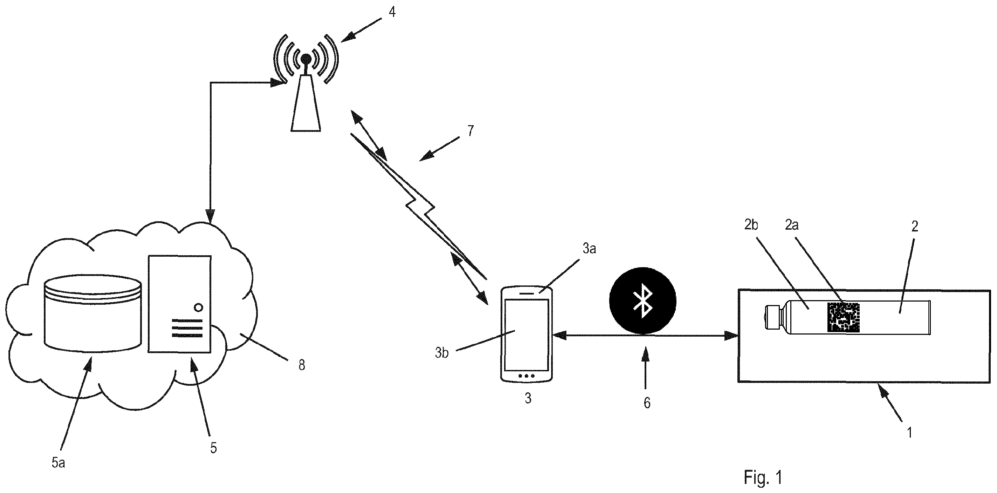

Ein erstes erfindungsgemässes Verabreichungssystem ist in Figur 1 gezeigt. Schematisch ist das Verabreichungsgerät 1 gezeigt, bei welchem es sich insbesondere um eine Infusionspumpe, spezielle eine Insulinpumpe 1 handeln kann. Solche Pumpen sind dem Fachmann in Form der mylife Ypsopump der Firma Ypsomed oder der 670G der Firma Medtronic hinlänglich bekannt und bedürfen deshalb keiner ausführlicheren Beschreibung. Wichtig ist, dass das Verabreichungsgerät 1 über eine Funkverbindung 6, speziell eine Bluetoothverbindung 6, bi-direktional mit einem mobilen Kommunikationsgerät 3, insbesondere einem Smart Phone 3, kommunizieren kann. In das Verabreichungsgerät 1 kann das wechselbare Reservoir 2 eingesetzt werden. Das Reservoir 2 kann wie gezeigt die Form einer Karpule haben, andere Formen sind wie erwähnt jedoch möglich. In diesem Beispiel ist die erfindungsgemässe Information in Form eines GS 1 DataMatrix Codes 2a auf der äusseren Oberfläche 2b der Karpule 2 angeordnet. Dabei kann der DataMatrix Code 2 direkt auf die Aussenwand 2b gedruckt sein, oder in Form oder als Teil einer Etikette auf die Aussenwand 2b angebracht, insbesondere geklebt, sein. Alternativ kann sie den Umfang der Aussenwand umschliessen und zum Beispiel aufgeschrumpft sein.

Das Smartphone 3 umfasst, wie bei Smart Phones üblich, eine oder mehrere Kamera an Vorder-und/oder Rückseite (symbolisch Bezugszeichen 3a). Auf dem Smart Phone 3 ist ein Betriebssystem, wie iOS oder Android, installiert, welches die Installation und das Ausführen der erfindungsgemässen Software-Applikation erlaubt. Das Smart Phone verfügt zumindest über ein Bluetooth- Modul, zur Bi-direktionalen Kommunikation mit Verabreichungsgerät 1, sowie über ein WLAN- und ein Mobilfunk-Modul, um via Internet und/oder via Mobilfunk (beides symbolische als Mobilfunkmast 4 dargestellt) in Verbindung mit einem entfernten Server 5 zu treten. Der Server 5 kann wie erwähnt in verschiedenen Varianten ausgestaltet sein. In der gezeigten Variante ist er Teil eines Cloud-Services 8, und kann als virtuelle Serverinstanz ausgestaltet sein. Dem Server zugeordnet ist eine Datenbank 5a, welche entweder direkt auf dem Server 5 läuft oder auf einer anderen Serverinstanz der Cloud 8 laufen kann.

Kamera 3b des Smart Phones 3 kann beim Reservoirwechsel genutzt werden um die Information vom Reservoir abzufotografieren. Im vorliegenden Beispiel handelt sich um einen genormten GS 1 DataMatrix Code, welcher verschiedene Daten zum Medikament enthält. Die Global Trade Item Number (GTIN), welche das Produkt eindeutig identifiziert (natürlich sind alternative Systeme möglich, die Kennung muss einfach eindeutig sein), eine Chargennummer, ein Verfallsdatum und optional eine Seriennummer und/oder eine Herstellerkennung. Weitere Daten könnten problemlos hinzugefügt werden und wären auch normkonform.

Nach dem Abfotografieren können die im DataMatrix Code codierten Daten aus der Fotografie im Smart Phone extrahiert und ausgewertet werden. Dazu stellen die Betriebssysteme iOS und Android Funktionen bereit, welche durch die Software-Applikation genutzt werden können. Alternativ können auch andere, auch separate Apps für die Datenextraktion genutzt werden, welche die extrahierten Daten nach Extraktion an die Software-Applikation übergeben.

Die Software-Applikation nutzt die Daten, um zu prüfen, welche Funktionalität der Software-Applikation mit der in den extrahierten Daten vorhandenen Information vereinbar ist. Die oben erwähnte in den Daten enthaltene GTIN identifiziert das Produkt eindeutig (zu prüfen z. B. unter compendium.ch), so identifiziert die GTIN 7680553460037 eindeutig Lantus von Sanofi in der Konzentration von 100 Einheiten/ml in 3 ml Karpulen (5er Packung). Das gleiche Medikament in derselben Konzentration in der gleichen Reservoirgrösse, aber im Solostar Fertigpen (5er Packung) hat die GTIN 7680580440019. Schnellwirksames Insulin Lispro von Eli Lilly in derselben Konzentration auch in 5x3ml Packung hat die GTIN 7680535530369. Während eine Verwechslung von Karpule und Fertigpen nicht zu erwarten ist, besteht jedoch die Gefahr, dass ein Patient statt eine Lantuskarpule (langsam wirksames Basalinsulin) eine Lispro (schnell wirksames Insulin) verwendet.

Wenn nun die Information mit dem Smart Phone 3 ausgelesen wird und die GTIN isoliert ist, kann die GTIN benutzt werden, um zu prüfen, ob der Patient das richtige Medikament in das Verabrei-chungsgerät, in diesem Fall eine Insulinpumpe, einsetzen will.

Im Beispiel sendet die Software-Applikation des Smart Phones 3 die extrahierte GTIN und allenfalls noch weitere Informationen einen entfernten Server, was über WLAN/Internet oder Mobilfunk/Internet 7 und 4 geschieht. Der Server 5 nimmt die GTIN entgegen und prüft über die Datenbank, ob es sich um ein zugelassenes Produkt handelt und wenn ja, um welches. Um beim Beispiel von Lantus/Lispro zu bleiben, kann es sein, dass die Insulinpumpe für Lispro von Eli Lilly empfohlen und zugelassen ist, während Lantus von Sanofi nicht zugelassen ist. Entspricht die GTIN nun Lispro von Lilly in der richtigen Konzentration und dem richtigen Gebinde, kann der Server entsprechend eine Antwort via Internet an das Smart Phone senden, welches zum Beispiel so dann die gesamte Funktionalität der Software Applikation freigibt. Wird umgekehrt eine ungültige GTIN oder ein für die Insulinpumpe nicht zugelassenes Insulin festgestellt kann der Server dies der Software-Applikation ebenfalls weiter vermitteln. Die Software-Applikation wird sodann nur eine sehr rudimentäre Funktionalität freigeben. Vorteilhaft zeigt die Software-Applikation eine Warnmeldung auf dem Display des Smart Phones, welche den Patienten auf das Problem aufmerksam macht. In einer weiteren Konsequenz kann die Software-Applikation ein Steuersignal an das Verabreichungsgerät, also die Insu-linpumpe des Beispiels, senden und dieses temporär blockieren. Will der Patient trotz aller Warnung und temporärer Blockierung das Medikament trotzdem einsetzen, so soll er das können. In der App steht ihm dann nur eine eingeschränkte Funktionalität zur Verfügung, zum Beispiel kann die Fernsteuerfunktionalität deaktiviert werden. Auf der Pumpe, kann der Patient die Blockierung manuell lösen.

Es ist wichtig, dass der Patient oder die Patientin bewusst das System umgehen kann. Es kann zum Beispiel sein, dass es bei einem bestimmten zugelassenen Medikament einen Lieferengpass gibt und kurzfristig ein alternatives Medikament eingesetzt werden muss, um eine Krankheit zu behandeln. So etwas muss weiter möglich sein, es soll jedoch vermieden werden, dass versehentlich ein falsches Medikament eingesetzt wird.

Das erste erfindungsgemässe System aus Figur 1 kann variiert werden, ohne vom Erfindungsgedanken abzuweichen. So ist zum Beispiel denkbar, dass die Software-Applikation auf dem Smart Phone, die Produktreferenzen (z. B. die GTIN) von allen zugelassenen Produkten in einer internen Datenablage hinterlegt und die Datenablage mit dem externen Server von Zeit zu Zeit abgeglichen wird.

Eine weitere Funktion, welche die Sicherheit erhöhen kann ist folgende: Zusätzlich zur Produktidentifikation kann auch die Chargennummer mit dem Server abgeglichen werden. Tritt zum Beispiel der Fall ein, dass eine Charge zurückgerufen werden muss und diese Information ist auf dem Server hinterlegt, dann kann der Server 5 in seiner Antwort auch hinterlegen, dass das Medikament zwar das richtige wäre, die Charge jedoch nicht genutzt werden sollte. Vorteilhaft kann die Software-Applikation eine entsprechende Meldung auf dem Display der Smart Phones 3 anzeigen, insbesondere zusammen mit Anweisungen, was der Patient oder die Patientin mit dem Medikament machen soll (z. B. eine Rückgabe an die nächste Apotheke).

Wie erwähnt ist vorteilhaft, wenn in der Information auch ein Verfallsdatum hinterlegt ist, so kann das Smart Phone 3 nach der Extraktion der Informationen über die intern vorhandene Uhr und den Kalender überprüfen, ob ein Medikament schon abgelaufen ist. Ist das der Fall, so stehen wieder verschiedene Möglichkeit für Konsequenzen im Raum: Deaktivierung von Funktionalität, Meldung an Patient oder Patientin via Display, temporäres Blockieren des Verabreichungsgeräts.

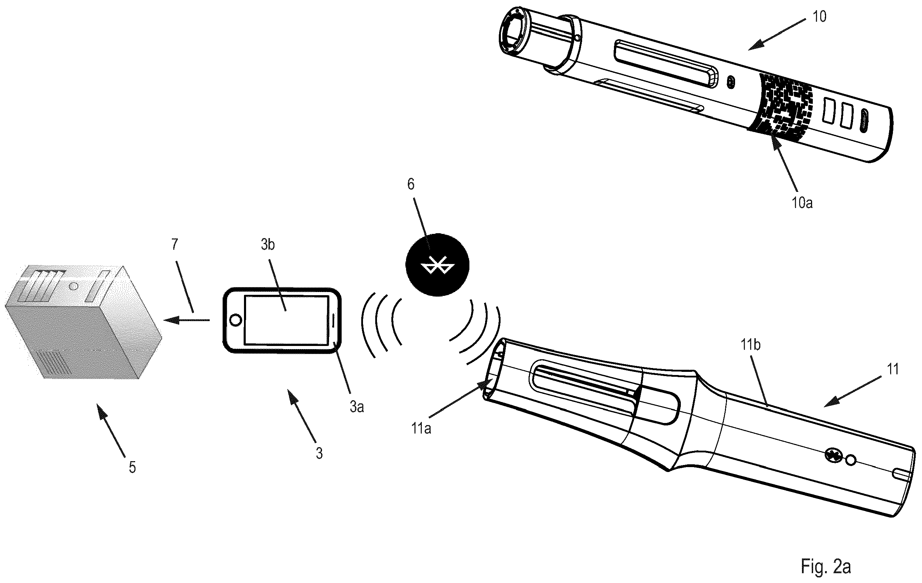

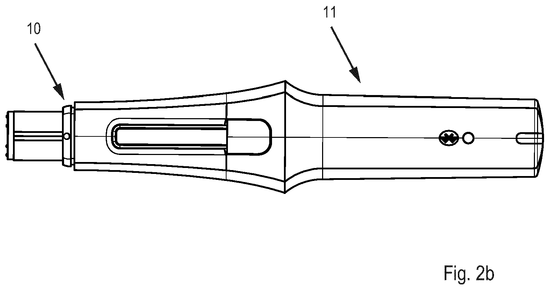

Ein zweites erfindungsgemässes System ist in den Figuren 2a und 2b dargestellt. Das Verabreichungsgerät besteht in diesem System aus zwei Komponenten einem Add-on 11 und einem Injektionsgerät 10. Beim gezeigten Injektionsgerät handelt es sich um einen Autoinjektor. Das Verabreichungsgerät aus dem gezeigten Add-on und dem Autoinjektor ist ausführlich in der WO 2018064784 A1 beschrieben, welche hiermit vollständig durch Verweis in das vorliegende Dokument aufgenommen ist.

Das Add-on 11 ist mehrfach verwendbar, während der Autoinjektor 10 für genau eine Injektion ausgelegt ist. Der Autoinjektor 11 wird also nach jeder Verabreichung ausgetauscht. Add-on und Autoinjektor werden für den Gebrauch miteinander mechanisch verbunden. Dazu wird der Autoinjektor in die Öffnung des Add-on 11 eingeschoben. Vorteilhaft wird der Autoinjektor 10 über einen mechanischen Schnappmechanismus im Add-on 11 gehalten.

Add-on 11 umfasst eine elektronische Steuerung und eine damit verbundene drahtlose Kommunikationsvorrichtung, im vorliegenden handelt es sich um Bluetooth. Die elektronische Steuerung kann via Bluetooth bi-direktional mit dem Smart Phone 3 kommunizieren. Das Add-on umfasst weiter Sensoren, die dazu dienen Zustände im Autoinjektor 10 zu detektieren, wenn Add-on 11 und Autoinjektor zusammengebaut sind. Es wird bezüglich Details explizit auf die oben zitierte WO 2018064784 A1 verwiesen. Die Zustände können zum Beispiel folgende Zustände beinhalten: ,vor der Injektion', ,während der Injektion', ,nach der Injektion'. Die detektierten Zustände können sodann an das Smart Phone 3 weitergegeben werden. Dieses Zustände können in der Software-Applikation protokolliert und weiter genutzt werden, zum Beispiel um dem Patienten oder der Patientin sinnvolle Informationen zur Nutzung des Medikaments, wie eine Anleitung, auf dem Display des Smart Phones einzublenden.

Der abgebildete Autoinjektor 10 enthält ein Reservoir mit einem fluiden Medikament und eine Ausschüttvorrichtung, welche eine automatische Injektion und Entleerung des Reservoirs nach einer Aktivierung der Ausschüttvorrichtung ermöglicht.

Wie bei Autoinjektoren üblich, dient als Reservoir eine Standard 1 ml Fertigspritze. Alternativ wäre auch eine 2.25 ml Variante möglich. Da es sich um weiteverbreitete Standardformen von Reservoirs handelt, kann der Baugleiche Autoinjektor 10 für sehr unterschiedliche zu injizierende Medikamente zum Einsatz gelangen, zum Beispiel zur Behandlung von rheumatoider Arthritis oder sexueller Dysfunktion. Entsprechend wichtig ist, dass keine Verwechslungen stattfinden.

Auf einer äusseren Oberfläche des Autoinjektors ist Information, beim vorliegenden Beispiel in Form eines GS 1 DataMatrix Codes 10a angeordnet. Wie beim ersten beschriebenen System enthält dieser GS 1 DataMatrix Code 10a eine eindeutige Identifikation des Medikamentes.

Wenn nun der Autoinjektor 10 am Add-on 11 ausgetauscht wird, kann eine Kamera des Smart Phones 3 dazu verwendet werden, den GS 1 DataMatrix Code 10a auszulesen und die Informationen analog zum ersten beschriebenen System zu prüfen. Fällt die Prüfung positiv aus, kann die Software Applikation auf dem Display das korrekte Medikament bestätigen und den Patienten oder Patientin via Display weiter Anleiten, zum Beispiel zeigen, wie der Autoinjektor 10 korrekt in das Add-on eingeführt wird. Nach dem Einführen, kann die Software-Applikation auf den vom Add-on 11 gemeldeten Zuständen weiter anleiten, bestätigen und protokollieren.

Weitere Anwendungen wie Überprüfung des Verfalldatums sind natürlich auch in diesem System möglich und erfindungsgemäss.

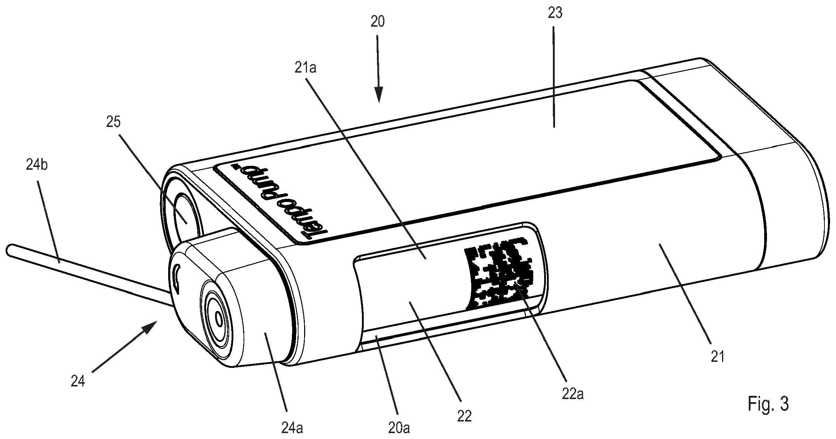

Ein drittes erfindungsgemässes System ist eine Variante des ersten beschriebenen erfindungsgemässen Systems. Sofern hier nicht erwähnt ist das dritte System gleich wie das erste. Figur 3 zeigt deshalb, um das vorliegende Dokument möglichst kompakt zu halten, nur das Verabreichungsgerät, nämlich die Insulinpumpe 20, des Systems. In Abwandlung handelt es sich beim Verabreichungsgerät 1 um eine Insulinpumpe 20, das Reservoir 2 mit dem Data Matrix Code 2a ist nun das Reservoir 22 mit dem Data Matrix Code 22a. Die Pumpe umfasst weiter ein Touch Display 23, ein Infusionsset 24 (wobei nur der Infusionssetadapter 24a und der Schlauch 24b abgebildet sind), sowie den Bedi-enknopf 25. Bei der Pumpe 20 handelt es sich um eine Ausführung der Ypsopump, welche via Touchscreen und Bedienknopf zu bedienen ist. Die Ypsopump ist in der WO2015/127965A1 ausführlich beschrieben. Die WO2015/127965A1 ist hiermit durch Verweis vollständig in das vorliegende Dokument aufgenommen.

Die Insulinpumpe 20 umfasst ein Gehäuse 21 mit einem Fenster 21a. Das Fenster 21a kann als transparenter Bereich des Gehäuses 21 ausgebildet sein. Alternativ kann das Gehäuse 21 am Ort des Fensters 21a eine Öffnung umfassen, in welche ein transparentes Stück Kunststoff oder Glas eingesetzt ist. Das Fenster 21a erlaubt die Sicht in das Karpulenfach 20a der Pumpe 20. In Figur 3 ist die Karpule 22 in die Pumpe 20 eingesetzt. Durch das Fenster 21a ist die Karpule 22 mit dem Data Matrix Code 22a sichtbar.

In Abwandlung zum ersten beschriebenen System wird nun der Data Matrix Code 22a erst nach dem Einsetzen der Karpule 22 in die Pumpe 20 mit der Smart Phone Kamera 3a erfasst. Dabei wird nicht nur der Data Matrix Code 22a erfasst, sondern auch die ganze Pumpe 20. Die Weiterverarbeitung der Information aus dem Data Matrix Code 22a läuft analog zur Verarbeitung im ersten System mit allen möglichen Varianten wie weiter oben beschrieben.

Hinzukommt nun die Verarbeitung der Bildinformation der Pumpe 20. Erfindungsgemäss kann über Bilderkennungspakete von Android (ML Kit oder OpenCV) oder iOS (AR Kit, Vision oder OpenCV) auf dem Smart Phone 4, wie sie dem Fachmann bekannt sind und off zur Verfügung stehen, respektive auf dem Server 5 beispielsweise mittels OpenCV, TensorFlow, PyTorch oder Amazon Image Recognition, erkannt werden, ob es sich bei der verwendeten Pumpe um eine zugelassene Infusionspumpe, zum Beispiel die Insulinpumpe handelt. Entsprechend der erkannten Pumpe kann erfindungsgemäss Funktionalität aktiviert oder deaktiviert werden.

Weitere Anwendungen wie Überprüfung des Verfalldatums sind natürlich auch in diesem System möglich und erfindungsgemäss.

### BEZUGSZEICHENLISTE

- 1: Verabreichungsgerät
- 2: Reservoir
- 2a: Data Matrix Code (Information)
- 3: Smart Phone (mobiles Kommunikationsgerät)
- 3a: Kamera (symbolisch)
- 3b: Display (Bildschirm)
- 4: Mobilfunknetz/Internet
- 5: Server
- 5a: Datenbank
- 6: Bluetooth-Verbindung
- 7: WLAN- oder Mobilfunk Verbindung
- 8: Cloud
- 10: Autoinjektor (Injektionsgerät)
- 10a: Data Matrix Code (Information)
- 11: Add-on
- 11a: Öffnung
- 20: Insulinpumpe
- 20a: Karpulen
- 21: Gehäuse
- 21a: Fenster im Gehäuse
- 22: Reservoir (Karpule)
- 22a: Data Matrix Code (Information)
- 23: Berührungssensitives Display (Touch Display)
- 24: Infusionsset
- 24a: Infusionssetadapter
- 24b: Infusionsleitung
- 25: Bedienknopf

## Patentansprüche

1. Verabreichungssystem zur Verabreichung eines fluiden Medikaments zumindest bestehend aus einem Verabreichungsgerät (1), insbesondere einer Infusionspumpe oder einem Injektionsgerät, und einem mobilen Kommunikationsgerät (3), insbesondere einem Mobiltelefon oder einem Smart Phone, welche drahtlos miteinander bidirektional kommunizieren können, das mobile Kommunikationsgerät (3) umfassend:
a. eine elektronische Steuerung mit einem Prozessor und einem Speicher, welche die Ausführung von Software Applikationen ermöglicht,
b. eine mit dem Prozessor und dem Speicher verbundene Kommunikationsvorrichtung, welche der drahtlosen Kommunikation (6) mit dem Verabreichungsgerät (1) dient, insbesondere eine Bluetooth-Einheit,
c. eine mit dem Prozessor und dem Speicher verbundene weitere Kommunikationsvorrichtung, welche der Kommunikation (7) mit entfernten Servern (5) dient, insbesondere eine WLAN und/oder eine Mobilfunkeinheit,
d. eine Software-Applikation, welche im Speicher des mobilen Kommunikationsgeräts (3) abgelegt ist und ausgeführt werden kann, und welche mehrere Funktionen zur Kommunikation mit dem Verabreichungsgerät (1) sowie der Steuerung des Verabreichungsgeräts implementiert,
e. eine Kamera(3a), welche mit Prozessor und Speicher verbunden ist und welche durch die Software Applikation ansprechbar und steuerbar ist,
f. eine Benutzerschnittstelle (3b), welche die Interaktion dem mobilen Kommunikationsgeräts (3) und einer benutzenden Person ermöglicht, insbesondere ein berührungssensitiver Bildschirm und/oder eine Kombination von Bildschirm und weiteren Bedienelementen, und wobei die Benutzerschnittstelle (3b) insbesondere auch die Interaktion von benutzender Person und Software Applikation ermöglicht,
das Verabreichungsgerät (1) zumindest umfassend:
g. ein austauschbares Reservoir (2) für das zu verabreichende fluide Medikament, wobei auf einer äusseren Oberfläche (2b) oder der Verpackung des Reservoirs Information (2a), insbesondere in Form eines Data Matrix Codes, QR Codes oder Barcodes, angeordnet ist,
h. eine elektronische Steuerung und eine mit der elektronischen Steuerung verbundene drahtlose Kommunikationsvorrichtung, insbesondere eine Bluetooth-Einheit, zur drahtlosen und bidirektionalen Kommunikation mit dem mobilen Kommunikationsgerät (3),
i. eine Ausschüttvorrichtung, welche über die elektronische Steuerung gesteuert wird und mit welcher fluides Medikament aus dem Reservoir (2) in einzelnen Dosen, quasikontinuierlich oder kontinuierlich über eine Verabreichungsleitung, insbesondere ein Infusionsset oder eine Injektionsnadel, verabreichbar ist,
j. ein Gehäuse, welches das austauschbare Reservoir, Steuerung, Kommunikationsvorrichtung und Ausschüttvorrichtung zumindest teilweise umschliesst,
wobei
k. beim Austausch des Reservoirs (2), bei welchem ein gebrauchtes Reservoir (2) entfernt wird und ein neues Reservoir (2) eingesetzt wird, die Information (2a) auf dem neuen Reservoir (2) oder dessen Verpackung mit der Kamera (3a) des Kommunikationsgeräts (3) auslesbar ist,
**dadurch gekennzeichnet, dass**
1. ein Teil der mehreren Funktionen der Steuerung des Verabreichungsgeräts (1) der Software-Applikation durch das Auslesen und Auswerten der Information (2a) aktivierbar oder deaktivierbar sind.

2. Verabreichungssystem nach dem vorhergehenden Anspruch, wobei die Information (2a) zumindest eines oder mehrere der folgenden Elemente umfasst: eine eindeutige Produktkennung, eine Chargennummer, eine Seriennummer, ein Verfallsdatum, ein Herstelldatum oder eine Herstellerkennung.

3. Verabreichungssystem nach den vorhergehenden Ansprüchen, wobei es sich beim Verabreichungsgerät (1) um eine Insulinpumpe handelt.

4. Verabreichungssystem nach den vorhergehenden Ansprüchen, wobei es sich bei der Form der Information (2a) um einen DataMatrix Code nach ISO/IEC 15418:2016 handelt.

5. Verabreichungssystem nach dem vorhergehenden Anspruch, wobei die Information (2a) in der DataMatrix Codes folgende Informationen enthält:
a. eine nationale oder globale Artikelnummer, als NTIN oder GTIN,
b. eine Verfallsdatum,
c. eine Chargennummer, und optional
d. eine Seriennummer.

6. Ein Verabreichungssystem nach einem der Ansprüche 1 bis 5, wobei das Verabreichungsgerät (1) eine Infusionspumpe (20) ist, wobei durch die Kamera (3a) des Kommunikationsgeräts (3) nicht nur die Information (2a) auslesbar ist, sondern auch Bildinnformation zu Aussehen und Form der Infusionspumpe (20), wobei die Bildinformation entweder auf dem Kommunikationsgerät (3) oder den entfernten Servern (5) verarbeitbar ist, und das Resultat der Verarbeitung Einfluss auf die Aktivierbarkeit oder Deaktivierbarkeit der Funktionen der Steuerung des Verabreichungsgeräts (1) der Software-Applikation durch das Auslesen und Auswerten der Information (2a) hat.

7. Methode zur Freigabe von Funktionen in einem Verabreichungssystem gemäss einem der vorhergehenden Ansprüche 1-5, wobei die freizugebenden Funktionen aktivierbare Funktionen der Software Applikation betreffen und wobei die Methode zumindest folgende Schritte umfasst:
a. Lesen der Information (2a) durch die Kamera des mobilen Kommunikationsgeräts (3), Erzeugung von Bildinformationen und Zwischenspeichern der Bildinformation im Speicher,
b. Extraktion der Information (2a) aus den erzeugten Bildinformationen mit Hilfe des Prozessors und Zwischenspeichern der extrahierten Information (2a) im Speicher,
c. Übertragung von zumindest Teilen der extrahierten Information über die weitere Kommunikationsvorrichtung (7) an einen entfernten Server (5), welcher eine Datenbank (5a) zur Überprüfung der übertragenen extrahierten Information umfasst und eine Aufstellung von aktivierbaren Funktionen enthält,
d. Überprüfung der übertragenen extrahierten Information in der Datenbank (5a), wobei überprüft wird, ob die aktivierbaren Funktionen kompatibel mit der übertragenen extrahierten Information sind,
e. Erstellung einer Antwort auf dem Server (5) auf Basis der Überprüfung und senden der Antwort vom Server (5) an das mobile Kommunikationsgerät (3) über die weitere Kommunikationsvorrichtung (7), und
f. keine, teilweise, oder vollständige Freigabe der aktivierbaren Funktionen der Software Applikation auf dem mobilen Kommunikationsgerät (3).

8. Methode nach dem vorhergehenden Anspruch, wobei die Antwort des Servers (5) eine Liste mit den mit der übertragenen extrahierten Information kompatiblen Funktionen überträgt und nur die kompatiblen aktivierbaren Funktionen in der Software Applikation freigegeben werden.

9. Methode zur Freigabe von Funktionen in einem Verabreichungssystem gemäss 1 bis 5, wobei die freizugebenden Funktionen aktivierbare Funktionen der Software Applikation betrifft, wobei der Speicher des mobilen Kommunikationsgerätes (3) eine lokale Datenbank enthält, welche Daten zur Überprüfung der Information enthält und wobei die Methode zumindest folgende Schritte umfasst:
a. Lesen der Information (2a) durch die Kamera (3a) des mobile Kommunikationsgeräts (3)und Erzeugung von Bildinformationen und Zwischenspeichern der Bildinformation im Speicher,
b. Extraktion der Information (2a) aus den erzeugten Bildinformationen mit Hilfe des Prozessors und Zwischenspeichern der extrahierten Information (2a) im Speicher,
c. Überprüfung von zumindest Teile der extrahierten Informationen in der lokalen Datenbank, wobei überprüft wird, ob die aktivierbaren Funktionen kompatibel mit der übertragenen extrahierten Information sind, und
d. keine, teilweise, oder vollständige Freigabe der aktivierbaren Funktionen der Software Applikation auf Basis der Überprüfung.

10. Methode nach Anspruch 7 oder 9, wobei wenn die Überprüfung der übertragenen extrahierten Information auf dem Server (5) oder der extrahierten Information in der lokalen Datenbank ergibt, dass mindestens eine aktivierbaren Funktionen nicht kompatibel ist, keine der freizugebenden Funktionen freigegeben wird.

11. Methode nach einem der vorhergehenden Ansprüche 7 bis 10, wobei das mobile Kommunikationsgerät (3) weiter eine Uhr, welche die aktuelle Uhrzeit wiedergeben kann, und einen Kalender, welcher das aktuelle Datum wiedergeben kann, umfasst und die extrahierte Information zumindest ein Ablaufdatum umfasst und wobei die Methode weiter folgende Schritte umfasst:
a. Vergleich des Ablaufdatums mit dem aktuellen Datum,
b. Freigabe keiner der freizugebenden Funktionen falls der Vergleich ergibt, dass das Ablaufdatum schon erreicht oder überschritten ist.

12. Methode nach dem vorhergehenden Anspruch, wobei falls der Vergleich ergibt, dass das Ablaufdatum schon erreicht oder überschritten ist, die Software Applikation eine Meldung auf der Benutzerschnittstelle (3b) ausgibt, welche die benutzende Person darauf aufmerksam macht, das das Ablaufdatum erreicht ist und deshalb die freizugebenden Funktionen nicht freigeben werden.

13. Methode nach einem der Ansprüche 11 oder 12, wobei das Verabreichungsgerät (1) weiter über eine Benutzerschnittelle zur Steuerung des Verabreichungsgeräts verfügt und wobei falls der Vergleich ergibt, dass das Ablaufdatum schon erreicht oder überschritten ist, die Software Applikation einen Steuerungsbefehl über die Kommunikationsvorrichtung an das Verabreichungsgerät (1) sendet (6), mit welchem das Verabreichungsgerät (1) temporär deaktiviert wird, und eine benutzende Person das Verabreichungsgerät (1) manuell über die Benutzerschnittstelle des Verabreichungsgeräts (1) reaktivieren muss.

14. System nach einem der Ansprüche 1 oder 5 oder eine Methode nach einem der Ansprüche 7 bis 13, **dadurch gekennzeichnet, dass** die aktivierten Funktionen eine oder mehrere der folgenden Funktionen umfasst:
a. eine Fernsteuerungsfunktion, mit welcher über die Software Applikation ein Bolus Kommando an das Verabreichungsgerät (1) sendbar ist, wobei Bolus Grösse und der zeitliche Verlauf der Bolusabgabe in der Software Applikation programmierbar sind,
b. eine Fernsteuerungsfunktion, mit welcher über die Software Applikation ein Kommando an das Verabreichungsgerät (1) sendbar ist, mit welchem laufende Medikamentenverabreichungen gestoppt werden,
c. eine Fernsteuerungsfunktion, mit welcher über die Software Applikation ein Kommando an das Verabreichungsgerät (1) sendbar ist, mit welchem unterbrochene Medikamentenverabreichungen wieder gestartet werden,
d. eine Fernsteuerungsfunktion, mit welcher über die Software Applikation ein Kommando an das Verabreichungsgerät (1) sendbar ist, mit welchem Medikamentenverabreichungen gestartet werden oder
e. eine Fernsteuerungsfunktion, mit welcher über die Software Applikation ein Kommando an das Verabreichungsgerät (1) sendbar ist, mit welchem etwaige Medikamentenverabreichungen temporär vermindert werden.

## Claims

1. Administration system for administering a fluid medicament, comprising at least an administration device (1), in particular an infusion pump or an injection device, and a mobile communication device (3), in particular a mobile telephone or a smart phone, which are able to communicate bidirectionally with one another wirelessly, the mobile communication device (3) comprising:
a. an electronic controller with a processor and a memory, which enables the execution of software applications,
b. a communication device connected to the processor and the memory, which serves for wireless communication (6) with the administration device (1), in particular a Bluetooth unit,
c. a further communication device connected to the processor and the memory, which serves for communication (7) with remote servers (5), in particular a WLAN and/or a mobile radio unit,
d. a software application which is stored in the memory of the mobile communication device (3) and can be executed and which implements a plurality of functions for communication with the administration device (1) as well as the control of the administration device,
e. a camera (3a) which is connected to the processor and the memory and which can be addressed and controlled by the software application,
f. a user interface (3b) which enables interaction between the mobile communication device (3) and a user, in particular a touch-sensitive screen and/or a combination of a screen and further operating elements, and wherein the user interface (3b) in particular also enables the interaction of the user and the software application,
the administration device (1) comprising at least:
g. an exchangeable reservoir (2) for the fluid medicament to be administered, wherein information (2a), in particular in the form of a Data Matrix code, QR code or barcode, is arranged on an outer surface (2b) or the packaging of the reservoir,
h. an electronic controller and a wireless communication device connected to the electronic controller, in particular a Bluetooth unit, for wireless and bidirectional communication with the mobile communication device (3),
i. a dispensing device which is controlled via the electronic controller and by means of which fluid medicament from the reservoir (2) can be administered in individual doses, quasi-continuously or continuously via an administration line, in particular an infusion set or an injection needle,
j. a housing which at least partially encloses the exchangeable reservoir, controller, communication device and dispensing device,
wherein
k. when the reservoir (2) is exchanged, in which a used reservoir (2) is removed and a new reservoir (2) is inserted, the information (2a) on the new reservoir (2) or its packaging can be read out with the camera (3a) of the communication device (3), **characterized in that**
l. a portion of the plurality of functions of the control of the administration device (1) of the software application are activatable or deactivatable by reading out and evaluating the information (2a).

2. Administration system according to the preceding claim, wherein the information (2a) comprises at least one or more of the following elements: a unique product identifier, a batch number, a serial number, an expiry date, a manufacturing date or a manufacturer identifier.

3. Administration system according to the preceding claims, wherein the administration device (1) is an insulin pump.

4. Administration system according to the preceding claims, wherein the form of the information (2a) is a DataMatrix code according to ISO/IEC 15418:2016.

5. Administration system according to the preceding claim, wherein the information (2a) in the DataMatrix codes contains the following information:
a. a national or global article number, as NTIN or GTIN,
b. an expiry date,
c. a batch number, and optionally
d. a serial number.

6. An administration system according to any of claims 1 to 5, wherein the administration device (1) is an infusion pump (20), wherein by means of the camera (3a) of the communication device (3) not only the information (2a) can be read out, but also image information on the appearance and shape of the infusion pump (20), wherein the image information can be processed either on the communication device (3) or the remote servers (5), and the result of the processing has an influence on the activatability or deactivatability of the functions of the control of the administration device (1) of the software application by reading out and evaluating the information (2a).

7. Method for releasing functions in an administration system according to any of the preceding claims 1-5, wherein the functions to be released relate to activatable functions of the software application and wherein the method comprises at least the following steps:
a. reading the information (2a) by the camera of the mobile communication device (3), generating image information and temporarily storing the image information in the memory,
b. extracting the information (2a) from the generated image information by means of the processor and temporarily storing the extracted information (2a) in the memory,
c. transmitting at least parts of the extracted information via the further communication device (7) to a remote server (5), which comprises a database (5a) for verifying the transmitted extracted information and contains a listing of activatable functions,
d. verifying the transmitted extracted information in the database (5a), wherein it is verified whether the activatable functions are compatible with the transmitted extracted information,
e. creating a response on the server (5) on the basis of the verification and sending the response from the server (5) to the mobile communication device (3) via the further communication device (7), and
f. no, partial, or complete release of the activatable functions of the software application on the mobile communication device (3).

8. Method according to the preceding claim, wherein the response of the server (5) transmits a list with the functions compatible with the transmitted extracted information and only the compatible activatable functions are released in the software application.

9. Method for releasing functions in an administration system according to 1 to 5, wherein the functions to be released relate to activatable functions of the software application, wherein the memory of the mobile communication device (3) contains a local database which contains data for verifying the information and wherein the method comprises at least the following steps:
a. reading the information (2a) by the camera (3a) of the mobile communication device (3) and generating image information and temporarily storing the image information in the memory,
b. extracting the information (2a) from the generated image information by means of the processor and temporarily storing the extracted information (2a) in the memory,
c. verifying at least parts of the extracted information in the local database, wherein it is verified whether the activatable functions are compatible with the transmitted extracted information, and
d. no, partial, or complete release of the activatable functions of the software application on the basis of the verification.

10. Method according to claim 7 or 9, wherein if the verification of the transmitted extracted information on the server (5) or of the extracted information in the local database reveals that at least one activatable function is not compatible, none of the functions to be released is released.

11. Method according to any of the preceding claims 7 to 10, wherein the mobile communication device (3) further comprises a clock which can reproduce the current time and a calendar which can reproduce the current date and the extracted information comprises at least an expiry date and wherein the method further comprises the following steps:
a. comparing the expiry date with the current date,
b. releasing none of the functions to be released if the comparison reveals that the expiry date has already been reached or exceeded.

12. Method according to the preceding claim, wherein if the comparison reveals that the expiry date has already been reached or exceeded, the software application outputs a message on the user interface (3b) which draws the user's attention to the fact that the expiry date has been reached and therefore the functions to be released are not released.

13. Method according to any of claims 11 or 12, wherein the administration device (1) further has a user interface for controlling the administration device and wherein if the comparison reveals that the expiry date has already been reached or exceeded, the software application sends a control command to the administration device (1) via the communication device (6), by which the administration device (1) is temporarily deactivated, and a user must reactivate the administration device (1) manually via the user interface of the administration device (1).

14. System according to any of claims 1 or 5 or a method according to any of claims 7 to 13, **characterized in that** the activatable functions comprise one or more of the following functions:
a. a remote control function by means of which a bolus command can be sent to the administration device (1) via the software application, wherein bolus size and the temporal course of the bolus delivery are programmable in the software application,
b. a remote control function by means of which a command can be sent to the administration device (1) via the software application by which ongoing medicament administrations are stopped,
c. a remote control function by means of which a command can be sent to the administration device (1) via the software application by which interrupted medicament administrations are started again,
d. a remote control function by means of which a command can be sent to the administration device (1) via the software application by which medicament administrations are started, or
e. a remote control function by means of which a command can be sent to the administration device (1) via the software application by which any medicament administrations are temporarily reduced.

## Revendications

1. Système d'administration destiné à l'administration d'un médicament fluide, comprenant au moins un dispositif d'administration (1), en particulier une pompe à perfusion ou un dispositif d'injection, et un dispositif de communication mobile (3), en particulier un téléphone mobile ou un smartphone, qui peuvent communiquer l'un avec l'autre de manière bidirectionnelle et sans fil, le dispositif de communication mobile (3) comprenant :
a. une commande électronique avec un processeur et une mémoire, qui permet l'exécution d'applications logicielles,
b. un dispositif de communication relié au processeur et à la mémoire, qui sert à la communication sans fil (6) avec le dispositif d'administration (1), en particulier une unité Bluetooth,
c. un autre dispositif de communication relié au processeur et à la mémoire, qui sert à la communication (7) avec des serveurs distants (5), en particulier une unité WLAN et/ou de radiocommunication mobile,
d. une application logicielle qui est stockée dans la mémoire du dispositif de communication mobile (3) et peut être exécutée, et qui met en œuvre plusieurs fonctions pour la communication avec le dispositif d'administration (1) ainsi que la commande du dispositif d'administration,
e. une caméra (3a) qui est reliée au processeur et à la mémoire et qui peut être sollicitée et commandée par l'application logicielle,
f. une interface utilisateur (3b) qui permet l'interaction entre le dispositif de communication mobile (3) et une personne utilisatrice, en particulier un écran tactile et/ou une combinaison d'un écran et d'autres éléments de commande, et l'interface utilisateur (3b) permettant en particulier également l'interaction de la personne utilisatrice et de l'application logicielle,
le dispositif d'administration (1) comprenant au moins :
g. un réservoir interchangeable (2) pour le médicament fluide à administrer, une information (2a), en particulier sous la forme d'un code Data Matrix, d'un code QR ou d'un code-barres, étant disposée sur une surface externe (2b) ou sur l'emballage du réservoir,
h. une commande électronique et un dispositif de communication sans fil relié à la commande électronique, en particulier une unité Bluetooth, pour la communication sans fil et bidirectionnelle avec le dispositif de communication mobile (3),
i. un dispositif de distribution qui est commandé via la commande électronique et au moyen duquel un médicament fluide du réservoir (2) peut être administré en doses individuelles, quasi-continûment ou continûment via une ligne d'administration, en particulier un set de perfusion ou une aiguille d'injection,
j. un boîtier qui entoure au moins partiellement le réservoir interchangeable, la commande, le dispositif de communication et le dispositif de distribution,
k. lors de l'échange du réservoir (2), au cours duquel un réservoir usagé (2) est retiré et un nouveau réservoir (2) est inséré, l'information (2a) sur le nouveau réservoir (2) ou son emballage pouvant être lue à l'aide de la caméra (3a) du dispositif de communication (3), **caractérisé en ce que**
l. une partie des plusieurs fonctions de la commande du dispositif d'administration (1) de l'application logicielle sont activables ou désactivables par lecture et évaluation de l'information (2a).

2. Système d'administration selon la revendication précédente, dans lequel l'information (2a) comprend au moins un ou plusieurs des éléments suivants : un identifiant de produit unique, un numéro de lot, un numéro de série, une date de péremption, une date de fabrication ou un identifiant de fabricant.

3. Système d'administration selon les revendications précédentes, dans lequel le dispositif d'administration (1) est une pompe à insuline.

4. Système d'administration selon les revendications précédentes, dans lequel la forme de l'information (2a) est un code DataMatrix selon ISO/IEC 15418:2016.

5. Système d'administration selon la revendication précédente, dans lequel l'information (2a) dans les codes DataMatrix contient les informations suivantes :
a. un numéro d'article national ou mondial, en tant que NTIN ou GTIN,
b. une date de péremption,
c. un numéro de lot, et facultativement d. un numéro de série.

6. Système d'administration selon l'une quelconque des revendications 1 à 5, dans lequel le dispositif d'administration (1) est une pompe à perfusion (20), dans lequel, au moyen de la caméra (3a) du dispositif de communication (3), non seulement l'information (2a) peut être lue, mais aussi une information d'image relative à l'aspect et à la forme de la pompe à perfusion (20), l'information d'image pouvant être traitée soit sur le dispositif de communication (3), soit sur les serveurs distants (5), et le résultat du traitement ayant une influence sur l'activabilité ou la désactivabilité des fonctions de la commande du dispositif d'administration (1) de l'application logicielle par lecture et évaluation de l'information (2a).

7. Procédé de libération de fonctions dans un système d'administration selon l'une quelconque des revendications précédentes 1-5, dans lequel les fonctions à libérer concernent des fonctions activables de l'application logicielle et dans lequel le procédé comprend au moins les étapes suivantes :
a. lecture de l'information (2a) par la caméra du dispositif de communication mobile (3), génération d'une information d'image et mémorisation temporaire de l'information d'image dans la mémoire,
b. extraction de l'information (2a) à partir de l'information d'image générée à l'aide du processeur et mémorisation temporaire de l'information extraite (2a) dans la mémoire,
c. transmission d'au moins une partie de l'information extraite via l'autre dispositif de communication (7) à un serveur distant (5), lequel comprend une base de données (5a) pour la vérification de l'information extraite transmise et contient un inventaire de fonctions activables,
d. vérification de l'information extraite transmise dans la base de données (5a), où il est vérifié si les fonctions activables sont compatibles avec l'information extraite transmise,
e. création d'une réponse sur le serveur (5) sur la base de la vérification et envoi de la réponse du serveur (5) au dispositif de communication mobile (3) via l'autre dispositif de communication (7), et
f. absence, libération partielle ou libération complète des fonctions activables de l'application logicielle sur le dispositif de communication mobile (3).

8. Procédé selon la revendication précédente, dans lequel la réponse du serveur (5) transmet une liste comprenant les fonctions compatibles avec l'information extraite transmise et seules les fonctions activables compatibles sont libérées dans l'application logicielle.

9. Procédé de libération de fonctions dans un système d'administration selon 1 à 5, dans lequel les fonctions à libérer concernent des fonctions activables de l'application logicielle, dans lequel la mémoire du dispositif de communication mobile (3) contient une base de données locale qui contient des données pour la vérification de l'information et dans lequel le procédé comprend au moins les étapes suivantes :
a. lecture de l'information (2a) par la caméra (3a) du dispositif de communication mobile (3) et génération d'une information d'image et mémorisation temporaire de l'information d'image dans la mémoire,
b. extraction de l'information (2a) à partir de l'information d'image générée à l'aide du processeur et mémorisation temporaire de l'information extraite (2a) dans la mémoire,
c. vérification d'au moins une partie de l'information extraite dans la base de données locale, où il est vérifié si les fonctions activables sont compatibles avec l'information extraite transmise, et
d. absence, libération partielle ou libération complète des fonctions activables de l'application logicielle sur la base de la vérification.

10. Procédé selon la revendication 7 ou 9, dans lequel, si la vérification de l'information extraite transmise sur le serveur (5) ou de l'information extraite dans la base de données locale révèle qu'au moins une fonction activable n'est pas compatible, aucune des fonctions à libérer n'est libérée.

11. Procédé selon l'une quelconque des revendications précédentes 7 à 10, dans lequel le dispositif de communication mobile (3) comprend en outre une horloge, qui peut restituer l'heure actuelle, et un calendrier, qui peut restituer la date actuelle, et l'information extraite comprend au moins une date de péremption, et dans lequel le procédé comprend en outre les étapes suivantes :
a. comparaison de la date de péremption avec la date actuelle,
b. absence de libération de l'une quelconque des fonctions à libérer si la comparaison révèle que la date de péremption a déjà été atteinte ou dépassée.

12. Procédé selon la revendication précédente, dans lequel, si la comparaison révèle que la date de péremption a déjà été atteinte ou dépassée, l'application logicielle émet un message sur l'interface utilisateur (3b) attirant l'attention de la personne utilisatrice sur le fait que la date de péremption est atteinte et que, de ce fait, les fonctions à libérer ne sont pas libérées.

13. Procédé selon l'une quelconque des revendications 11 ou 12, dans lequel le dispositif d'administration (1) comporte en outre une interface utilisateur pour la commande du dispositif d'administration, et dans lequel, si la comparaison révèle que la date de péremption a déjà été atteinte ou dépassée, l'application logicielle envoie une commande de commande au dispositif d'administration (1) via le dispositif de communication (6), par laquelle le dispositif d'administration (1) est temporairement désactivé, et une personne utilisatrice doit réactiver manuellement le dispositif d'administration (1) via l'interface utilisateur du dispositif d'administration (1).

14. Système selon l'une quelconque des revendications 1 ou 5 ou procédé selon l'une quelconque des revendications 7 à 13, **caractérisé en ce que** les fonctions activables comprennent une ou plusieurs des fonctions suivantes :
a. une fonction de télécommande, au moyen de laquelle une commande de bolus peut être envoyée au dispositif d'administration (1) via l'application logicielle, la taille du bolus et le déroulement temporel de l'administration du bolus étant programmables dans l'application logicielle,
b. une fonction de télécommande, au moyen de laquelle une commande peut être envoyée au dispositif d'administration (1) via l'application logicielle, par laquelle des administrations de médicament en cours sont arrêtées,
c. une fonction de télécommande, au moyen de laquelle une commande peut être envoyée au dispositif d'administration (1) via l'application logicielle, par laquelle des administrations de médicament interrompues sont relancées,
d. une fonction de télécommande, au moyen de laquelle une commande peut être envoyée au dispositif d'administration (1) via l'application logicielle, par laquelle des administrations de médicament sont démarrées, ou
e. une fonction de télécommande, au moyen de laquelle une commande peut être envoyée au dispositif d'administration (1) via l'application logicielle, par laquelle d'éventuelles administrations de médicament sont temporairement réduites.
